# EUROPEAN PATENT APPLICATION

(11) **EP 2 255 811 A1**
(43) Date of publication of application: **01.12.2010**
(21) Application number: 10177151.7
(22) Date of filing: 04.12.2008
(51) Int. Cl.: A61K 31/5415, A61P 17/00, A61P 17/04

(54) **Mequitazine for treating or preventing pathologies involving histamine H4 receptors**

(30) Priority: 04.12.2007 FR 0759538; 23.06.2008 FR 0854144
(62) Divisional of application: 08856761.5
(71) Applicant: PIERRE FABRE MEDICAMENT, 92100 Boulogne-Billancourt (FR)
(72) Inventor: Clerc, Thierry, 31320, VIGOULET AUZIL (FR); Przybylski, Christophe, 31290, VILLEFRANCHE DE LAURAGAIS (FR); Tisne-Versailles, Jacky, 81100, CASTRES (FR); Cussac, Didier, 81100, CASTRES (FR)
(74) Representative: Ahner, Francis

(57) **Abstract**

The present application relates to the use of 10-[(3R)-1-azabicyclo[2.2.2]oct-3-ylmethyl]-10*H*-phenothiazine, 10-[(3S)-1-azabicyclo[2.2.2]oct-3-ylmethyl]-10*H*-phenothiazine or 10-[(3R,3S)-1-azabicycio[2.2.2]oct-3-ylmethyl]-10H-phenothiazine or a pharmaceutically acceptable salt thereof, to prepare a drug to prevent or treat pathologies involving histamine H4 receptor, chosen among atopic dermatitis and pruritus.

## Description

The present invention relates generally to the use of 10-[(3R,3S)-1-azabicyclo[2.2.2]oct-3-ylmethyl]-10H-phenothiazine, 10-[(3S)-1-azabicyclo[2.2.2]oct-3-ylmethyl]-10*H*-phenothiazine or 10-[(3R)-1-azabicycio[2.2,2]oct-3-ylmethyl]-10*H*-phenothiazine as well as pharmaceutically acceptable salts thereof to prepare a drug to prevent or treat, by local, oral or pulmonary route, pathologies mediated by activation of histamine H4 receptor. 10-[(3R)-1-azabicyclo[2.2.2]oct-3-ylmethyl]-10*H*-phenothiazine and 10-[(3S)-1-azabicycio[2.2.2]oct-3-ylmethyl]-10*H*-phenothiasine derive from a racemate. To begin with, this racemate is a drug effective in the treatment of allergic disorders. It is an antihistamine of the H1 class. It has an asymmetrical carbon that leads to two distinct spatial configurations: levorotatory (configuration S) and dextrorotatory (configuration R). Analysis of the pharmacological profiles of the two enantiomers and the racemic mixture shows that these compounds, in an unexpected way, have affinity for histamine H4 receptor. This property is in addition to their affinity for H1 receptor. This particular property has not been demonstrated or simply even considered in the prior art. It is exemplified below.

H4 receptor, described by Nakamura et al. (2000) and cloned by Morse et al. (2001), is a transmembrane protein of 390 amino acids coupled with the Gi/o heterodimer, whose activation induces intracellular calcium mobilization, a decrease in cAMP and activation of the MAP kinase pathway. Although this receptor is three-dimensionally similar to H1 receptor, they are only 35% homologous.

It is widely postulated that activation of one or another histamine receptor subtype would be expressed by different physiological or physiopathological effects. This receptor selectivity would justify the existence of histamine receptor subtypes. Activation of H1 receptor induces activation of protein Gq, that of H2 receptor induces activation of protein Gs, that of H3 receptor induces activation of protein Gi/o (Lovenberg et al., 1999). Functionally, activation of these receptors is expressed by a different molecular effect. Depending on the H3 receptor ligands considered, some have an affinity for H4 receptor. These include thioperamide, clobenpropit, imetit and R-α-methylhistamine. Moreover, the distribution of receptor subtypes is also different. For humans, H4 receptor is primarily expressed in peripheral tissues such as bone marrow cells and hematopoietic cells (eosinophils, basophils, mast cells, T lymphocytes and dendritic cells).

Apart from their anatomical distribution, the specificity of the role of histamine receptors in physiopathology depends on the involvement of histamine in these processes. The function of histamine as an allergy mediator is well established. Much recent data attributes to histamine a function in the regulation of the immune response, more precisely via recently described H4 receptor. Receptor H4 antagonists inhibit the response of T lymphocytes, and the cellular immune response to antigen stimulation is inhibited *in vitro* by inactivation of H4 receptor according to an apoptotic protein-kinase dependent process (Sugata et al., 2007). Histamine is an important mediator for the immediate allergy response but it also intervenes in the regulation of antigen-presenting cells (Dy et al., 2004). Thus, activation of H4 receptor induces monocyte differentiation in immunocompetent dendritic cells. When H4 receptor is activated, a decrease in IL-12 p70 appears and leads to monocyte differentiation (Gutzner et al., 2005). Takeshita et al. (2003) showed that H4 receptor intervened directly in the recruitment of polynuclear neutrophils, this recruitment or chemotaxis being dependent on mast cells.

Thus, the decrease in chemotaxis and thus the decrease in neutrophil infiltration via H4 receptor inactivation has been clearly established. For example, in models of inflammation caused by injection of agents such as carrageenans, or in models of inflammation resulting from ovalbumin challenge, the importance of H4 receptor antagonists emerges as putative agents for controlling peripheral inflammatory phenomena. The majority are related to a physiopathology:
- atopic dermatitis, dermatological pruritus,
- bronchial inflammations (asthma, obstructive bronchopneumopathy),
- intestinal inflammation (irritable bowel syndrome),
- articular inflammation (rheumatoid arthritis),
- inflammation of the peritumoral stroma allowing angiogenesis and its consequence: tumor development.

### Involvement of H4 receptor in dermatological inflammation / pruritus

Inhibition of H4 receptor by a specific ligand such as JNJ7777120 limits self-scratching behaviors in animals in which pruritus was induced beforehand by subcutaneous histamine injection (Bell et al., 2004). It appears that the activity of H4 receptor antagonists is higher than that of H1 antagonists such as desloratadine or cetirizine. However, the activity of these "classical" antihistamines is not zero in this model, thus showing that the antipruritic effect of H1 antihistamines does not lie in their sedative property. Moreover, this antipruritic activity was shown for certain H1 antagonists stripped of sedative effects. In addition, the peripheral localization of H4 receptor confirms the hypothesis according to which its inactivation enables local control of the affection, as opposed to central control. Receptor H4 expression corroborates these data. Indeed, according to Lippert et al. (2004), human cutaneous mast cells express histamine H4 receptor, which confirms the role of histamine as an autocrine and paracrine regulator of cutaneous inflammation. Moreover, it has been shown that in T lymphocytes from patients with no atopy, histamine via H4 receptor acts by inhibiting STAT1 protein activation. This messenger is a protein that activates transcription of factors controlling the immune response and Th1/Th2 balance. In the case of lymphocytes from patients with atopy, the signal is still mediated by the activation of H4 receptor by histamine and is finally expressed by the generation of interferon-γ. This factor causes an amplification of inflammatory phenomena. In cells from patients with no atopy, the expression of STAT1 is already reduced and the level of interferon-γ is low. Thus, inhibition of H4 receptor is all the more effective among patients with atopy than among healthy volunteers (Horr et al., 2006).

It can thus be concluded that the combined effect of an H1 antagonist coupled with an H4 antagonist would constitute a property of choice for a therapeutic agent intended to limit the dermatological consequences of pruritus, such as itching and its associated complications (lesions, infections).

### Involvement of H4 receptor in airway inflammation

Expression of H4 receptor in the bronchi and the alveolar epithelium led the investigations to study its involvement in bronchial inflammatory phenomena such as asthma or obstructive bronchopneumopathy. Animal models of these pathologies using ovalbumin challenge made it possible to specify the role of H4 receptor in this context (Dunford et al., 2006). As seen previously, inactivation of H4 receptor via specific antagonists limits chemotaxis and thus neutrophil infiltration within the inflamed pulmonary epithelium. The consequence of this is a disruption of the immune and inflammatory cascade. Indeed, CD4 T-lymphocytes are no longer activated when H4 receptor is neutralized. Moreover, chemokine and cytokine levels decrease, the Moreover, chemokine and cytokine levels decrease, the consequence being a decrease in T lymphocyte response. Thus, it is logical to propose any H4 receptor antagonist as an agent to fight inflammatory phenomena of the pulmonary epithelium, whatever its origin: intoxication, allergic reaction or chronic respiratory distress induced by a tumor.

### Involvement of H4 receptor in intestinal tract inflammation

Another example of inhibition of inflammatory phenomena by H4 receptor antagonists is provided in the studies of Varga et al. (2005). Inflammation of the colon is induced in the animal by administration of trinitrobenzene. Beforehand, a treatment using H4 receptor antagonists is applied. In the end, the animals thus pretreated have lower histological inflammation scores than the control 1 animals. In parallel, a decrease is noted in levels of inflammation factors such as TNF-α. Thus, the importance of H4 receptor inhibitors appears in pathologies such as irritable bowel syndrome.

### Involvement of H4 receptor in articular inflammation

The involvement of H4 receptor has also been established in inflammatory phenomena consecutive to rheumatoid arthritis. Ikawa et al, (2005) analyzed the expression of H4 receptor by the indirect measurement of transcripts (PT-PCR) in samples of synoviocytes from patients with rheumatoid arthritis. The analyses show that the expression of H4 receptor is very high in these samples, suggesting that histamine H4 receptor plays a role in the inflammatory cascade consecutive to rheumatoid arthritis. Consequently, the importance of H4 receptor inhibitors in inflammatory pathologies of synoviocytes appears certain.

### Involvement of H4 receptor in angiogenesis associated with tumor development

The activity of histidine decarboxylase, an enzyme that catalyzes histamine synthesis, is found in a number of tumor tissues. This does not necessarily mean that the increase in histamine levels causes the appearance of carcinomatous phenomena, but it is, however, clearly established that the inflammation concomitant with tissue cancerization involves histamine. Histidine decarboxylase is expressed in tumors of the colon, breast and endometrium, small cell lung tumors and in melanomas. Work by Cianchi et al. (2005) demonstrated the overexpression of this enzyme in Caco2, HT29 and HCT116 cells. *In vitro* histamine induced in these cultures of adenocarcinoma of the colon an increase in the production of pro-inflammatory factors, namely prostaglandin E2, and an increase in the production of vascular endothelial growth factors (VEGF). It is probable that this agonist effect of histamine via H4 receptor is expressed by potentiation of cell division. Indeed, these two biomarkers participate in the development of the peritumoral vascular network (angiogenesis). The agonist action of histamine on vascular development is antagonized by H4 receptor inhibitors as well as by cyclooxygenase 1 and 2 inhibitors. Although the action of COX inhibitors was known because of their anti-inflammatory potential, the action of H4 receptor antagonists and their effectiveness on peritumoral vascular network development phenomena are newly described. These data suggest a usefulness of H4 receptor antagonists in angiogenesis. Moreover, the results of these studies show that inhibition of histamine activity by blocking H4 receptors decreases cell division by colon adenocarcinoma cells in culture.

For the reasons cited above, it appears that the candidate inhibitor should have a dual binding action on both H1 receptor and H4 receptor in order to have a complete action with respect to inhibition of histamine-mediated peripheral inflammation.

Within the context of dermatological pathologies involving H4 receptor, and with the aim of decreasing the adverse effects induced by antihistamines, the transdermal route appears advantageous since the candidate molecule has a propensity to cross the corneal layer and to reach its subcutaneous target, the mast cells expressing H4 receptors. Similarly, within the context of inflammatory pathologies of the airways, it is advantageous to provide a galenical form likely to reach the target directly in the bronchial epithelium by limiting the systemic penetration of the agent of interest.

Thus, the limitation of plasma levels of active metabolites of drugs can also rest, beyond a specific route of administration, on a particular hepatic metabolism that does not produce active metabolites capable of exacerbating the effect of the initially administered drug or increasing the frequency and intensity of side effects.

Moreover, longer product half-life means lower administration frequency, which facilitates treatment compliance.

The object of the present invention is to demonstrate the particular and unexpected properties of 10-[(3R)-1-azabicyclo[2.2.2]oct-3-ylmethyl]-10*H-*phenothiazine (dextrorotatory enantiomer of mequitazine; codified here under the name V0162), 10-[(3S)-1-azabicyclo[2.2.2]oct-3-ylmethyl]-10H-phenothiazine (levorotatory enantiomer of mequitazine; codified here under the name V0114) and the racemic mixture of the two enantiomers 10-[(3R,3S)-1-azabicyclo[2.2.2]oct-3-ylmethyl]-10*H*-phenothiazine (codified here under the name L0013).

The present invention relates to the use of 10-[(3R)-1-azabicyclo[2.2,2]oct-3-ylmethyl]-10*H-*phenothiazine, 10-[(3S)-1-azabicyclo[2.2.2]oct-3-ylmethyl]-10*H*-phenothiazine or 10-[(3R,3S)-1-azabicyclo[2.2.2]oct-3-ylmethyl]-10*H*-phenothiazine, or a pharmaceutically acceptable salt thereof, to prepare a drug to prevent or treat pathologies involving histamine H4 receptors.

According to another characteristic of the invention, the pathologies involving histamine H4 receptors are chosen among: atopic dermatitis, pruritus and bronchial inflammations such as asthma and obstructive bronchopneumopathy.

According to another characteristic of the invention, the drug is provided in a form suitable for oral administration comprising a sufficient content of active ingredient to antagonize target histamine H4 receptors.

According to another characteristic of the invention, the drug is provided in an oral dosage unit enabling an administration dose between 1 µg/kg and 10 mg/kg, advantageously between 0,01 mg/kg and 1 mg/kg.

According to another characteristic of the invention, the drug is provided in tablet form.

According to another characteristic of the invention, the drug is provided in the form of an oral spray solution or a powder for inhalation, specifically suited to treat asthma or obstructive bronchopneumopathy.

According to another characteristic of the invention, the drug is used to prevent or treat pathologies involving histamine H4 receptors and muscarinic receptors simultaneously.

According to another characteristic of the invention, the pathologies involving the aforementioned receptors are chosen among the respiratory pathologies.

According to another characteristic of the invention, the aforementioned pathologies are chosen among emphysema, asthma and obstructive bronchopneumopathy.

According to another characteristic of the invention, the invention extends to a product containing 10-[(3R)-1-azabicyclo[2.2.2]oct-3-ylmethyl]-10*H*-phenothiazine.

According to another characteristic of the invention, the drug is provided in a form suited to its administration by topical route.

According to another characteristic of the invention, the drug is provided as a gel, emulsion or cream with a concentration of active ingredient between 0,01% and 10% by weight.

According to another characteristic of the invention, the drug is used to treat atopic dermatitis and pruritus.

Atopic dermatitis is an inflammatory disease of the skin, sometimes called atopic eczema, characterised by pruriginous lesions of various origins, for example, bacterial, fungal or allergenic.

Generally, the object of the present invention is to treat pruritus, which is generally regarded as a symptom of itching skin appearing in connection with various types of dermatological lesions,

Lastly, the invention also extends to a product containing 10-[(3R)-1-azabicyclo[2.2.2]oct-3-ylmethyl]-10*H*-phenothiazine, 10-[(3S)-1-azabicyclo[2.2.2]oct-3-ylmethyl]-10*H*-phenothiazine or 10-[(3R,3S)-1-azabicyclo[2.2.2]oct-3-ylmethyl]10*H*-phenothiazine, or a pharmaceutically acceptable salt thereof, as well as a drug of the class of H1 antihistamines, corticoids, long- or fast-acting antimuscarinics and β2- mimetics, as a combination product for simultaneous, separate or extended use in an anti-inflammatory therapy by antagonis, of histamine H4 receptors.

The examples below show that:
- *in vitro* affinity for human histamine H4 receptor is effective at micromolar concentrations of the three compounds cited above.
- the compounds behave as inverse agonists with respect to H4 receptor, this property having never been described in the prior art.
- the transcutaneous or transmucosal passage of compounds V0114, V0162 and L0013 is considerable and is specific to this pharmacophore.
- the half-life of these compounds is long, which makes it possible to envisage dosing schedules compatible with the potential indications for these products.

The combination of all these properties, gathered as examples of the present patent, shows in an unexpected way that 10-[(3R)-1-azabicyclo[2.2.2]oct-3-ylmethyl]-10*H*-phenothinazine (dextrorotatory enantiomer; codified here under the name V0162), 10-[(3S)-1-azabicyclo[2.2.2]oct-3-ylmethyl]-10*H*-phenothiazine (levorotatory enantiomer; codified here under the name V0114) and the racemic mixture of the two enantiomer 10-[(3R,3S)-1-azabicyclo[2.2.2]oct-3-ylmethyl]-10*H-*phenothiazine (codified here under the name L0013) are compounds active in the pathologies involving H4 receptors, such as those listed above.

### EXAMPLE 1: In vitro affinity to human histamine H4 receptors by 10-[(3R)-1-azabicyclo[2.2.2]oct-3-ylmethyl]-10H-phenothiazine (dextrorotatory enantiomer; codified here under the name V0162), 10-[(3S)-1-azabicyclo[2.2.2]oct-3-ylmethyl]-10H-phenothiazine (levorotatory enantiomer; codified here under the name V0114) and the racemic mixture of the two enantiomers 10[(3R,3S)-1-azabicyclo[2.2.2]oct-3-ylmethyl]-10H-phenothiazine (codified here under the name L0013)

The goal of this study is to determine an affinity constant of the compound with respect to human histamine H4 receptor *in vitro*. The model chosen is HEK-293 cells transfected in a stable fashion by cDNA coding for human H4 receptor (Liu et al., 2001). First is determined the affinity of cells expressing each type of receptor for a ligand for which it was also established that it bound 100% to the receptor. The optimal ligand for the recombinant receptor is 10 nM tritiated histamine. Receptor binding is defined as follows: the difference between total binding and the nonspecific binding determined in the presence of excess cold ligand. The results are expressed as a percentage of optimal binding obtained with the ligand model (100%).

**Table 1: Receptor binding in vitro**

| Human receptor | Compounds tested at 10 µM | Code | Inhibition (%) |
|---|---|---|---|
| H4 | (10-[(3S)-1-azabicyclo[2.2.2]oct- | V0114 | 33 |
| (*h*) | 3ylmethyl]-10*H*-phenothiazine) (10-[(3R)-1-azabicyclo[2.2.2]oct-3-ylmethyl]-10*H*-phenotiazine) (10-[(3R,3S)-1-azabicyclo[2.2.2]oct-3-ylmethyl]-10*H*-phenothiazine | V0162 | 52 |

These results show that V0162, V0114 and L0013 are compounds that, in an unexpected way, strongly bind to human histamine H4 receptors.

In a second series of experiments, the affinity constants of these three compounds are precisely determined by competition with the tritiated histamine radioligand on membrane preparations from CHO cells expressing in a stable manner histamine H4 receptor.

**Table 2: Determination of affinity constants with respect to H4 receptor in vitro**

| Code | Compounds tested | Kᵢ (µM) |
|---|---|---|
| V0114 | (10-[(3S)-1-azabicyclo[2.2.2]oct-3-ylmethyl]-10*H*-phenothiazine) | 2,49±0,37 |
| V0162 | (10-[(3R)-1-azabicyclo[2.2.2]oct-3-ylmethyl]-10*H*-phenothiazine) | 1,18±0,16 |
| L0013 | (10-[(3R,3S)-1-azabicyclo[2.2.2]oct-3-ylmethyl]-10*H*-phenothiazine) | 1,63±0,20 |

These results show that the three compounds exhibit affinities with respect to histamine H4 receptor. These affinities are on the micromolar level. The activity of these compounds with respect to H4 receptors is reinforced by the fact that the three compounds V0162, V0114 and L0013 have a high capacity to cross the cutaneous barrier. Moreover, their long half-life in the organism suggests a residual effect on the level of the receptor target.

### EXAMPLE 2: Characterization of compounds V0162, V0114 and L0013 as inverse agonists of H4 receptor

These studies were carried out with the aim of defining the functional impact on H4 receptor (agonist, antagonist, inverse agonist) of compounds 10-[(3R)-1-azabicyclo[2.2.2]oct-3-ylmethyl]-10*H*-phenothiazine (dextrorotatory enantiomer; codified here under the name V0162), 10-[(3S)-1-azabicyclo[2.2.2]oct-3-ylmethyl]-10*H*-phenothiazine (levorotatory enantiomer; codified here under the name V0114) and the racemic mixture of the two enantiomers 10-[(3R,3S)-1-azabicyclo[2.2.2]oct-3-ylmethyl]-10H-phenothiazine (codified here under the name L0013).

G proteins belong to a family of proteins that act as molecular switches in numerous essential cell functions. Their regulatory activity is based on their capacity to cycle between an inactive form related to GDP and an active form related to GTP, the latter transmitting an action signal to so-called effector proteins located downstream in the signal cascade. In order to characterize the functional impact of the three compounds V0162, V0114 and L0013 on H4 receptor, studies using the G protein activation test were carried out. Briefly, the test rests on the quantification of [³⁵S] radiolabeled GTP bound to G proteins induced during binding of the various compounds on the receptor.

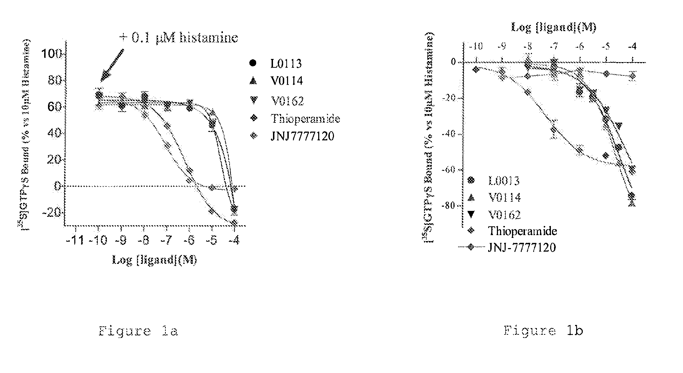

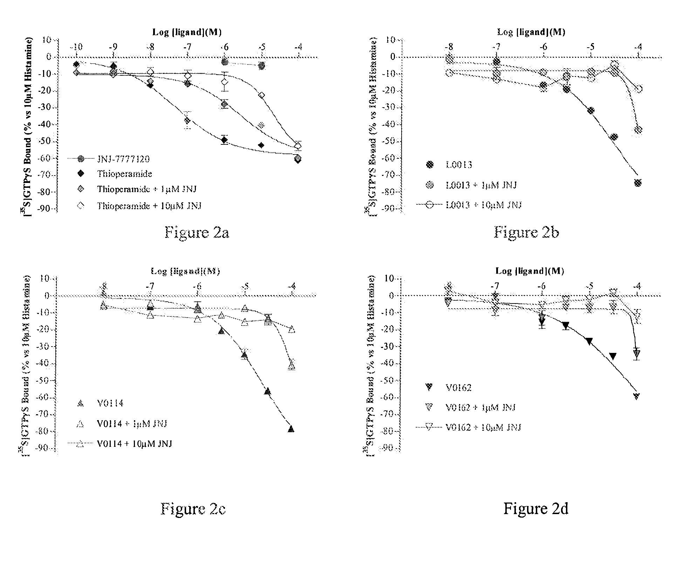

These results are reported in the form of diagrams presented in annexed figures 1 and 2.

The results of the G protein activation test presented in figure 1a show that the three compounds V0114, V0162 and L0013 antagonize the activating action induced by 0,1 µM histamine on H4 receptor, as observed with thioperamide and selective H4 antagonist JNJ7777120. H4 receptor is known to have a constitutive activity and figure 1b shows that compounds V0114, V0162 and L0013 behave like thioperamide as inverse agonists of H4 receptor (decrease in basal activity in the absence of agonist), whereas compound JNJ7777120 is a neutral antagonist.

This inverse agonist activity of compounds V0162, V0114, L0013 and thioperamide is certainly due to action on H4 receptor because figures 2a to 2d show that neutral antagonist JNJ7777120 blocks this activity. The totality of these results show for the first time that compounds V0162, V0114 and L0013 are inverse agonists of H4 receptor. This functioning on receptor inactivation differentiates V0162, V0114 and L0013 from the family of neutral antagonists such as JNJ7777120.

### EXAMPLE 3: Anti-inflammatory activity in vivo of V0114 and L0013 in the model of plantar edema induced by histamine in the mouse

The goal of this experiment was to detect possible anti-inflammatory activity of V0114, V0162 and L0013 with respect to plantar edema induced by histamine in the mouse. The principle of the test is inspired by the publication of Kreutner et al. (2000). All groups were composed of 10 animals. The products were administered by oral route (25 ml/kg) in pretreatment, at various times: 120, 90, 60 and 30 minutes before the inflammation-producing agent. Sixty minutes later, an injection (10 µl) in the paw was given under isoflurane anesthesia. The right paw received 13 µg of histamine dihydrochloride and the left paw physiological saline solution (histamine vehicle). Thirty minutes after the injections, the animals were euthanized by cervical dislocation. The paws were sampled and weighed. The edemas were quantified by the difference between the weights of the paws (weight of right paw weight of left paw).
Table 3: Determination of anti-inflammatory activity *in vivo*, after oral administration

**Table 4: Determination of antihistamine activity in vivo, after intravenous administration of the compounds**

| Compounds tested | Experimental group | Doses (mg/kg) | Pretreatment (min) | Inhibition of inflammation (%) |
|---|---|---|---|---|
| convey | 10 | - | -120 | - |
| | 10 | - | -90 | - |
| | 10 | - | -60 | - |
| | 10 | - | -30 | - |
| V0114 | 10 | 3 | -120 | -71 |
| | 10 | 3 | -90 | -72 |
| | 10 | 3 | -60 | -58 |
| | 10 | 3 | -30 | -63 |
| V0162 | 10 | 3 | -120 | +12 |
| | 10 | 3 | -90 | -5 |
| | 10 | 3 | -60 | +25 |
| | | | -30 | +2 |
| L0013 | 10 | 3 | -120 | -80 |
| | 10 | 3 | -90 | -82 |
| | 10 | 3 | -60 | -65 |
| | 10 | 3 | -30 | -54 |

V0114 and L0013 induce a powerful and statistically significant anti-inflammatory effect in comparison with the groups pretreated with vehicle at same times. This activity (-58% to -72%) for V0114 and (-54% to -82%) for L0013 is higher when the animals are pretreated 90 and 120 minutes before injection in the paw of the inflammation-producing agent. Under these experimental conditions, V0114 and L0013, administered by oral route 120, 90, 60 and 30 minutes beforehand, show a powerful anti-inflammatory effect with respect to histamine edema on the paw of the mouse. V0162 does not reduce this edema, which would tend to prove the absence of an antihistamine effect in this model. However, the products are administered by oral route. Yet in the following test, it appears that product V0162 is active when administered directly by intravenous route. In conclusion, this study shows that the H4 antihistamine activity demonstrated *in vitro* is confirmed in the animal *in vivo*.

### EXAMPLE 4: Antihistamine activity in vivo of V0114 and L0013 after administration by intravenous route, in the model of histamine-induced bronchoconstriction in the mouse

The goal of these experiments is to evaluate the activity of the three compounds V0162, V0114 and L0013 with respect to histamine-induced bronchoconstriction in the Konzett (1940) guinea pig model. Previously we saw that the three compounds exerted antihistamine activity with respect to H4 receptor *in vitro*, while on the other hand *in vivo* only V0114 and L0013 appeared anti-inflammatory when the inflammation is generated by histamine. It seems that V0162 loses its activity when it is administered by oral route. With an aim of analysing the activity by intravenous route of the three products, the following test was conducted. The animals are ventilated, by means of a constant pressure respiratory pump, by a volume of air in excess. The air, which does not penetrate into the lungs, arrives at a sensor and measures each inspiration. The variations observed represent changes in bronchial tonus. Histamine-induced bronchoconstriction causes an increase in the excess volume measured by the sensor. The various compounds are administered by way of the jugular vein, which is catheterized beforehand. Histamine stimulation is then carried out by intravenous route at a dose of 7 µg/kg.

**Table 4: Determination of antihistamine activity in vivo, after intravenous administration of the compounds**

| Compounds tested | Experimental group | Doses (mg/kg) | Inhibition at 5 min (%) | Inhibition at 30 min (%) |
|---|---|---|---|---|
| vehicle | 8 | - | 0 | 0 |
| V0114 | 6 | 12,5 | -15±3 | -35+6 |
| | 5 | 25 | -29±3 | -71+5 |
| | 5 | 37,5 | -51±8 | -91+2 |
| | 5 | 50 | -48±6 | -97+2 |
| V0162 | 5 | 25 | -17+3 | -9±8 |
| | 5 | 37,5 | -23+4 | -9±9 |
| | 5 | 50 | -31+12 | -4±10 |
| L0013 | 6 | 12,5 | -35±6 | -39±7 |
| | 10 | 25 | -35±5 | -61±5 |
| | 6 | 37,5 | -26±4 | -51±8 |
| | 5 | 50 | -37±5 | -57±7 |

Histamine induces bronchoconstriction in this model. When administered by intravenous route, the three compounds induce inhibition of the effects of histamine. Thus the route of administration appears important for reaching the target. But this result is modified when measurements after 30 min are considered. V0162 appears to lose its antagonistic activity, whereas this activity is maximal with V0114,

These experiments show that, *in vivo*, after intravenous administration, the compounds oppose the action of histamine on the phenomena that generate this mediator on the pulmonary level.

### EXAMPLE 5: Transcutaneous passage of 10-[(3R,3S)-1-azabicyclo[2.2.2]oct-3-ylmethyl]-10H-phenothiazine

The importance of H4 receptor in pruritus was recently established. Thus, an antagonist targeting the histamine H4 receptors involved in dermatological pruritus can constitute a weapon of choice for breaking behaviors related to pruritus and, consequently, for avoiding superinfections generated by the skin lesions caused by pruritus. Similarly, such an H4 antihistamine would prove useful in preventing or eliminating the symptoms observed in cases of atopy, as shown previously. To this end, it is useful to have an H4 antihistamine likely to cross the cutaneous barrier in order to reach its target on subcutaneous mast cells. The goal of these experiments is to evaluate the capacity of 10-[(3R,3S)-1-azabicyclo[2.2.2]oct-3-ylmethyl]-10H-phenothiazine to cross the cutaneous barrier. Human skin explants were placed in a dynamic diffusion chamber (diffusion area of 0,636 cm²), with the dermis in contact with the receptor medium (water; 0,9% NaCl 0,9% NaN₃ 0,1% / ethanol; 75/25; v/v). The flow rate is 1,5 ml/h. The study is carried out at 32°C. The solution containing the compound (500 µl to 2,5 mg/ml, or 10 µCi per cell) is deposited at T=0. The study of transcutaneous passage is carried out under occlusion. The receiving liquid is sampled at 3, 6, 9, 12, 16, 20 and 24 hours.

The results show that a nearly constant maximum flow of the compound is reached after 16 hours, and the accumulated quantities at 24 hours are 5,4 µg/cm²/h; 107 µg/cm².

The amount of compound in the epidermis and dermis (fig. 3b) at 24 hours shows a concentration gradient and thus a homogeneous distribution of this molecule in the skin:
- Epidermis = 56±17 µg/cm²
- Dermis = 45±14 µg/cm²
- Total = 101 µg/cm²
- Receptor zone = 107±28 µg/cm²

This study shows that 10-[(3R,3S)-1-azabicyclo[2.2.2]oct-3-ylmethyl]-10H-phenothiazine is a molecule with a good potential for transcutaneous passage (approx. 100 µg/cm²/24 h). The quantity of compound stored in the skin, after 24 hours, is equivalent or even higher than the accumulated quantity found in the receiving liquid after 24 hours.

### EXAMPLE 6: Evaluation of anti-inflammatory activity of compounds V0114 and V0162 in the obstructive chronic bronchopneumopathy model in the rat

The importance of the compound in decreasing pulmonary inflammation was evaluated in the following model. The animals are exposed to doses of cadmium by chronic atomization. Eventually, the animals develop an inflammatory reaction and pulmonary emphysema. This model was validated in the rat for its use in identifying potentially effective molecules to treat chronic respiratory diseases related to the persistence of a neutrophilic inflammatory reaction mediated by H4 receptor activation. Once the pathology is in place, the animals are treated daily by pulmonary administration of doses varying between 10 µg and 50 µg of the compound of interest administered by atomization.

The results show that the compounds tested partially restore respiratory capacity, most notably respiration rate, and increase inspiratory and expiratory flow. Moreover, at the end of the experiment, the bronchoalveolar fluids are sampled and immunocompetent cells are counted. The results show a decrease in infiltrated neutrophils when the treated groups are compared with the animals having received only placebo.

The totality of these results confirms the anti-inflammatory activity of the compounds tested via antagonism of histamine H4 receptor.

The three compounds exhibit receptor affinities with respect to H4 receptor. We established that they are inverse agonists of this receptor. *In vitro* and *in vivo* these anti-inflammatory activities were shown and established in the accepted models. Moreover, these compounds have qualities of pharmacokinetics and transcutaneous penetration that allow topical administration (cutaneous or pulmonary). The particular properties of these three compounds on the H₄ receptor and the resulting therapeutic uses have never been demonstrated before. These properties, taken together or considered separately, are exemplified above.

These examples show the rational use of 10-[(3R)-1-azabicyclo[2.2.2]oct-3-ylmethyl]-10*H*-phenothiazine, 10-[(3S)-1-azabicyclo[2.2.2]oct-3-ylmethyl]-10*H-*phenothiazine or 10-[(3R,3S)-1-azabicyclo[2.2.2]oct-3-ylmethyl]-10*H*-phenothiazine in pathologies mediated by histamine H4 receptor. Moreover, the previously established activity of the levorotatory derivative (10-[(3S)-1-azabicyclo[2.2.2]oct-3-ylmethyl]-10*H-*phenothiazine) and the racemic mixture (10-[(3R,3S,)-1-azabicyclo[2.2.2]oct-3-ylmethyl]-10*H*-phenothiazine) as H1 receptor inhibitors confers on these two products a dual H4 and H1 antihistamine property. This dual capacity makes these compounds superior in comparison to the antihistamine products described to date in the prior art. Similarly, the antimuscarinic activity of the dextrorotatory derivative (10-[(3R)-1-azabicyclo[2.2.2]oct-3-ylmethyl]-10*H*-phenothiazine) clearly established elsewhere is reinforced by its H4 antihistamine activity. This dual property is not described for compounds (10-[(3R,3S,)-1-azabicyclo[2.2.2]oct-3-ylmethyl]-10*H*-phenothiazine), (10-[(3R)-1-azabicyclo[2.2.2]oct-3-ylmethyl]-10*H-*phenothiazine) and (10-[(3S)-1-azabicyclo[2.2.2]oct-3-ylmethyl]-10*H*-phenothiazine) in the prior art.

The present invention has in addition shown for V0162 and L0013 the importance of the dual anti-H4 and anti-M1/M3 activity in inhibiting inflammatory phenomena and respiratory distress among patients with asthma or obstructive bronchopneumopathy.

Receptor H4 is involved in pathologies involving the recruitment of immunocompetent cells, most notably eosinophils and macrophages. It was thus envisaged to use the ligands of this receptor with the aim of limiting eosinophil infiltrations and macrophage recruitment on the pulmonary level during emphysema, obstructive bronchopneumopathy or asthma. This activity can be judiciously supplemented by a spasmolytic activity.

In an unexpected way, V0162 or (10-[(3R)-1-azabicyclo[2.2.2]oct-3-ylmethyl]-10*H*-phenothiazine) and L0013 or (10-[(3R,3S)-1-azabicyclo[2.2.2]oct-3-ylmethyl]-10*H*-phenothiazine) demonstrate a complementary anticholinergic activity. This activity is not observed *in vivo* for the levorotatory enantiomer V0114 or (10-[(3S)-1-azabicyclo[2.2.2]oct-3-ylmethyl]-10*H*-phenothiazine).

These two properties combined in a single compound make it possible to envisage the use of V0162 and / or L0013 to treat respiratory pathologies such as asthma and obstructive bronchopneumopathy.

### EXAMPLE 7: Affinity of V0162 with respect to muscarinic receptors

The goal of this test is to evaluate the inhibitory activity of the compounds on the binding of acetylcholine on M1, M2 and M3 receptors. To this end, a fixed quantity of radiolabeled acetylcholine is incubated in the presence of variable doses of the compounds to test: V0162, L0013 and V0114.

| Human receptors | Compound tested | IC₅₀ (nM) | Kⱼ (nM) | n_{H} |
|---|---|---|---|---|
| M1 | V0114 | 5,9 | 5,1 | 1,1 |
| *(h)* | **V0162** | **1,6** | **1,4** | **1,0** |
| | L0013 | 2,1 | 1,8 | 0.8 |
| M2 (*h*) | V0114 | 94 | 66 | 1,0 |
| | **V0162** | **10** | **7** | **1.1** |
| | L0013 | 20 | 14 | 0,9 |
| M3 (*h*) | V0114 | 17 | 12 | 1,0 |
| | **V0162** | **5,5** | **3,9** | **1,1** |
| | L0013 | 8 | 5,7 | 1,2 |

Thus, *in vitro* V0162 and L0013 show a very high affinity with respect to the muscarinic receptors involved in initiating, regulating and maintaining bronchial 1 muscle contractions. The levorotatory enantiomer is less inhibiting of acetylcholine binding to the three muscarinic receptors. This difference is even more remarkable *in vivo,* since only V0162 and L0013 antagonize the effect of acetylcholine.

### EXAMPLE 8: Inhibition of the effect of acetylcholine on respiratory muscle contraction

Guinea pigs are anaesthetized and artificially ventilated. The air penetrating the lungs is measured by means of a sensor. Each inspiration is thus quantified. Bronchoconstriction is then induced by intravenous injection of acetylcholine. The effect of acetylcholine is a direct and immediate modification of bronchial tonus. The bronchoconstriction effects of acetylcholine are evaluated in mm at the peak of the effects. Twenty minutes after the injection of acetylcholine, the various products to test are administered: V0162, L0013 and V0114, The effects of the products are compared with those of atropine, the reference method.

| Compounds tested | Dose (µg/kg) | Percentage of inhibition (%) | n |
|---|---|---|---|
| vehicle | - | - | 6 |
| V0162 | 25 | **-9±3** | **5** |
| | 50 | **-29±4** | 5 |
| | 100 | **-37+5** | 5 |
| V0114 | 25 | **-7±3** | 5 |
| | 50 | **-6±1** | **5** |
| | 100 | **-14±4** | 5 |
| L0013 | 25 | **-8±1** | 5 |
| | 50 | **-25±2** | **5** |
| | 100 | **-24±6** | 5 |
| Atropine | 5 | **-76±8** | 5 |

The results show that V0162 and L0013 are two products capable of inducing inhibition of bronchospasms induced by acetylcholine. On the other hand, V0114 is not very active.

### EXAMPLE 9: Bronchodilator effect of L0013 absence of bronchodilator effect of V0114

The effects of V0114 (0,5, 50 and 100 mg/kg) on respiratory functions are analyzed after oral (per os) administration in male Wistar rats (8 rats / dose). L0013 (100 mg/kg) administered under the same conditions is used as a reference substance. Theophylline (100 mg/kg), administered under the same experimental conditions, is used as a reference substance. Carboxymethyl cellulose (0,5%) in distilled water is used as a control vehicle. At the end of the testing period (i.e., 360 minutes after administration of the dose), the behavior of the animals as well as the diameter of their pupils (miosis / mydriasis) are evaluated. Blood samples are taken from each rat (except those pertaining to the theophylline group) in order to analyze the concentration of the substance in the blood. The control vehicle does not have an effect on time of inspiration, expiration, peak flow of inspiration, of expiration, respiratory volume, respiratory rate, relaxation time or respiratory pause and improves respiratory pause after the 360 minute test period. A very slight increase was observed in respiratory pause following the administration (+24% maximum at 120 minutes in the control vehicle group, p<0,01). V0114 (0,5, 50 and 100 mg/kg, p.o.) does not have a significant effect on any of the nine respiratory parameters evaluated, contrary to the control vehicle group after the 360 minute test period.

L0013 (100 mg/kg, p.o.) significantly increases respiratory rate (+80% maximum, compared to -9% in the control vehicle group, at 60 minutes of the administration, p<0,05). L0013 tends to decrease time of inspiration (-26% compared to +6% in the control vehicle group, at 60 minutes of the administration), time of expiration (-24% compared to +3% in the control vehicle group, at 60 minutes of the administration), relaxation time (-30% compared to -2% in the control vehicle group, at 120 minutes of the administration) and respiratory volume (-26% compared to -6% in the control vehicle group, at 60 minutes of the administration), It also tends to increase the peak flow of inspiration (+14% compared to -13% in the control vehicle group, at 60 minutes of the administration), the peak flow of expiration (+23% compared to -4% in the control vehicle group, at 120 minutes of the administration). These effects indicate stimulatory and bronchodilatory respiratory properties.

Theophylline (100 mg/kg, p.o.) clearly and rapidly decreases time of inspiration (-60% compared to +6% in the control vehicle group, at 60 minutes of the administration, p<0,001), time of expiration (-63% compared to +3% in the control vehicle group, at 60 minutes of the administration, p<0,001) and relaxation time (-61% compared to +0% in the control vehicle group, at 60 minutes of the administration, p<0,001), Theophylline clearly and rapidly increases peak flow of inspiration (+116% compared to -13% in the control vehicle group, at 60 minutes of the administration, p<0,001), peak flow of expiration (+121% compared to -7% in the control vehicle group, at 60 minutes of the administration, p<0,001) and respiratory rate (+223% compared to -9% % in the control vehicle group, at 60 minutes of the administration, p<0,001). Moreover, theophylline slightly decreases respiratory pause (-20% compared to +15% in the control vehicle group, at 300 minutes of the administration, p<0,01), and improves spiratory pause (-37% compared to +19% in the control vehicle group, at 300 minutes of the administration, p<0,05). Theophylline also tends to slightly and gradually decrease respiratory volume (-17% compared to +2% in the control vehicle group, at. 120 minutes of the administration, NS). These effects show the stimulatory and bronchodilatory respiratory properties of theophylline.

At the end of the experiment (i.e., 360 minutes post-administration), no change of behavior or signs of morbid state were noted following administration of V0114 (0,5, 50 and 100 mg/kg), L0013 (100 mg/kg) and theophylline (100 mg/kg). In the group treated with V0114, 360 minutes after administration, rat pupil diameter was measured and an increase of 50 mg/kg related to the administration was noted. In an identical way, an appreciable increase was noted in pupil diameter of the rats treated with L0013 (100 mg/kg) used as a control substance.

These results suggest that V0114 (0,5, 50 and 100 mg/kg) orally administered in male Wistar rats does not have any effect on respiratory functions after the 360 minute test period.

On the other hand, L0013 (100 mg/kg) reveals effects attesting to its stimulatory and bronchodilatory respiratory properties.

Taken together, these results show that V0162 and L0013 demonstrate antimuscarinic potential in addition to their H4 receptor inhibition activity. These two properties are important to the aim of preparing drug forms to prevent, reduce and treat respiratory pathologies involving muscarinic and histaminergic H4 receptors such as obstructive bronchopneumopathy, asthma or any respiratory function deficit.

### EXAMPLE 10: Favorable effect of V0162 administered in solution or powder form inhalation on dyspnea in animals

Ethanol is used as a solvent to administer V0162 by atomization. In addition, an anhydrous powder formulation was prepared. Two galenical forms are evaluated in these experiments: the solution of V0162 and inhaled powder containing V0162 The rats are treated by atomization of a solution of V0162 (10-[(3R)-1-azabicycio[2.2.2]oct-3-ylmethyl]-10*H-*phenothiazine) or by pulverization of an anhydrous powder containing V0162. V0162 was administered at various concentrations, the animal being placed in an exposure chamber. Fifteen minutes after exposure to V0162, the methacholine stimulation test is conducted. The animals are treated with increasing concentrations of methacholine. The values presented are the values of resistance opposed by the respiratory system to the air flow after each methacholine administration step. The results show that V0162 decreases dyspnea and thus increases residual functional capacity.

Methacholine challenge test in the rat, effect of two inhaled formulations of V0162 (powder and solution).

| | Resistance opposed | | | |
|---|---|---|---|---|
| | Dose of agonist (Mg/ml) | Placebo | V0162 solution | V0162 ponder |
| Methacholine | 0 | 0 | 0 | 0 |
| | 4 | 1,5 | 0,2 | 0,3 |
| | 8 | 4,5 | 1,9 | 0,8 |
| | 16 | 8,2 | 1 | 0,8 |

The dual antimuscarinic and anti-inflammatory activity via the inhibition of H4 receptor seem to confer V0162, inhaled in the form of powder or solution, a genuine property that can be exploited in the treatment of bronchial inflammations such as obstructive bronchopneumopathy or asthma, and respiratory distress generally.

## Claims

1. Use of 10-[(3R)-1-azabicyclo[2.2.2]oct-3-ylmethyl]-10*H*-phenothiazine, 10-[(3S)-1-azabicyclo[2.2.2]oct-3-ylmethyl]-10*H*-phenothiazine or 10-[(3R,3S)-1-azabicyclo[2.2.2]oct-3-ylmethyl]-10*H-*phenothiazine, or a pharmaceutically acceptable salt thereof, to prepare a drug to prevent or treat pathologies involving histamine H4 receptor, chosen among: atopic dermatitis and pruritus.

2. Use according to claim 1, **characterized in that** the drug is 10-[(3S)-1-azabicyclo[2.2.2]oct-3-ylmethyl]-10*H*-phenothiazine.

3. Use according to claim 1, **characterized in that** the drug is 10-[(3R)-1-azabicyclo[2.2.2]oct-3-ylmethyl]-10*H*-phenothizine.

4. Use according to claim 1 or 2, **characterized in that** the drug is provided in a form suited to its administration by topical route.

5. Use according to claim 4, **characterized in that** the drug is provided as a gel, emulsion or cream with a concentration of active ingredient between 0,01% and 10% by weight.
